# EUROPEAN PATENT SPECIFICATION

(11) **EP 2 343 079 B1**
(45) Date of publication and mention of the grant of the patent: **25.03.2015**
(21) Application number: 10184235.9
(22) Date of filing: 07.02.2002
(51) Int. Cl.: C12M 3/06

(54) **CELL SUSPENSION PREPARATION DEVICE**
VORRICHTUNG ZUR ZELLSUSPENSIONSHERSTELLUNG
APPAREIL POUR LA PREPARATION DE SUSPENSIONS CELLULAIRES

(30) Priority: 07.02.2001 AU PR298901; 04.04.2001 US 281527 P
(43) Date of publication of application: 13.07.2011
(62) Divisional of application: 02709917.5
(73) Proprietor: Avita Medical Ltd, Melbourn Royston Hertfordshire SG8 6HB (GB)
(72) Inventor: Stoner, Marie, South Perth, WA 6151 (AU); Wood, Fiona, Wembley, WA 6913 (AU)
(74) Representative: Trueman, Lucy Petra

(56) References cited:
- EP-A- 0 444 270
- AU-A- 3 990 197
- US-A- 3 647 632
- US-A- 4 649 115
- US-A- 5 601 728
- US-A- 5 786 207
- US-A- 6 080 581
- Gramlich: "ReCell ein Erfahrungsbericht", , XP002633923, Retrieved from the Internet: URL:http://www.hagenmuehle.de/documents/Mi crosoftWord-RecellVeroffentlichungInternet _000.pdf [retrieved on 2011-04-21]
- "Recell patient leaflet", , XP002633924, Retrieved from the Internet: URL:http://www.absolutemakeover.com.au/rec ell.htm [retrieved on 2011-04-21]
- MULEKAR S V ET AL: "Treatment of vitiligo lesions by ReCell vs. conventional melanocyte-keratinocyte transplantation: a pilot study.", THE BRITISH JOURNAL OF DERMATOLOGY JAN 2008 LNKD- PUBMED:17927795, vol. 158, no. 1, January 2008 (2008-01), pages 45-49, XP002633925, ISSN: 0007-0963
- NAVARRO F A ET AL: "SPRAYED KERATINOCYTE SUSPENSIONS ACCELERATE EPIDERMAL COVERAGE IN A PORCINE MICROWOUND MODEL", JOURNAL OF BURN CARE AND REHABILITATION, MOSBY- YEARBOOK INC, US, vol. 21, no. 6, 1 November 2000 (2000-11-01), pages 513-518, XP009025226, ISSN: 0273-8481

## Description

### FIELD OF THE INVENTION

This invention relates to a simple, rapid and cost effective technique for grafting of cells, and in particular to a device for preparing a suspension of cells from a tissue sample obtained from a donor site and applying that suspension of cells to a recipient site.

### BACKGROUND ART

There are many methods of treating wounds known to those skilled in the art. For example, skin grafting techniques exist which aim to reconstruct the skin covering areas of the body where there is either damage or defects to the skin. In general, these types of grafts are classified according to their host-donor relationship and by their thickness. The most clinically applied graft is the autologous graft, whereby tissue is taken from one area of the body and applied to another area. The grafted tissue then develops a new blood supply and attaches to the underlying tissues.

There are several types of skin grafts presently used, including split-thickness, full-thickness grafts, and micro-grafting. Each of these graft types must be prepared using certain techniques, and each one has its inherent advantages and disadvantages. Split-thickness grafts often require considerable skill, time and expensive equipment to perform. Further, donor sites are painful, result in scarring and limit the area able to be covered. Although they may be more successful than full-thickness grafts, they are usually less cosmetically attractive. Full-thickness grafts require less skill or expensive equipment, and their cosmetic appearance is better than that of split-thickness grafts. However, full-thickness grafts do not "take" as well as split-thickness grafts. Micro-grafts are more easily accomplished and require no special instruments. However, their cosmetic appearance is not as good as other techniques, as the resulting scarring is unacceptable.

A variation to the above grafting techniques is the mesh graft, which is a type of split-thickness or full-thickness skin graft in which parallel rows of slits are cut in the tissue being treated. Some of the advantages of mesh grafts include: a greater coverage of the effected area, drainage of blood or serum from under the graft, and increased conformity of the graft to uneven recipient areas. This technique has been very successful, with 90 to 100 percent "take" when the grafts have been applied on healthy granulation beds.

An alternative to split-skin grafting is to form a blister under suction at a donor site and transplant to the recipient site. The production of blisters to treat wounds has been used since the 1960s. The blisters are produced by a suction device, such as Dermavac™, at a suction pressure of approximately 250-300 mmHg for 1-2 hours. The blisters are then cut off and placed on the wound. The healing time is around 10-14 days. There are several disadvantages of this method such as the amount of time required to prepare the graft is too long and the graft may not result in re-pigmentation of the area; or uneven pigmentation is common around the edges of the area of treatment.

Micro-grafting has become a more common approach for large area cover and involves the "snipping off' of a number of very small sections of tissue from a donor site and applying then to a dressing, which is in turn applied to the wound area.

The most advanced technology for the generation of a tissue *in vitro* is to culture epidermis. Cultured epithelial autografts (CEA) are an important adjunct in the coverage of burns and other situations in which large areas of the body's surface experience skin loss. There are many centers throughout the world with tissue culture facilities whose aim is to produce autologous epithelial grafts for use in a wide variety of applications. The usefulness and application of CEA is related to its ability to achieve confluent cells sheets suitable for grafting. This technique overcomes many of the disadvantages of the previous treatments described above. For example, cultured epithelial autografts reduce the demand for donor sites. However, these autografts are slow growing and require time for culturing of the grafts, which often exceeds the time of preparation of the recipient's sites.

Disclosed is a cellular suspension together with a method for preparing that suspension and the present invention provides a device for its preparation each of which seek to ameliorate one or more of the disadvantages associated with prior art grafting technology.

### SUMMARY OF THE INVENTION

Disclosed is a unique cell suspension and method for its preparation that is rapid, efficient and simple to prepare and apply. Also disclosed is a method for treating a patient using the unique cell suspension and the subject invention relates to an apparatus suitable for use in the method preparation. Use of the described device while not essential for practicing the method disclosed has been found to significantly reduce the complexity associated with the use of conventional grafting technology such as CEA.

Disclosed is a method for preparing a cell suspension suitable for application to a patient, which method comprises the steps of:
(a) subjecting a tissue sample including cells suitable for grafting to a patient, to at least a physical and or chemical dissociating means capable of dissociating cellular stratum in the tissue sample;
(b) removing the tissue sample from the presence of the tissue dissociating means used in step (a) and harvesting in the presence of a nutrient solution cells from the tissue sample, cells suitable for grafting on to a patient wherein the nutrient solution is (i) free of xenogenic serum, (ii) capable of maintaining the viability of the cells until applied to a patient and (iii) is suitable for direct application to a region on a patient undergoing tissue grafting; and
(c) filtering the cellular suspension produced according to step (b) to remove large cellular conglomerates.

Preferably the dissociating means is of a chemical nature such as an enzyme which is capable of disrupting cellular bonding like for example trypsin. Further, preferably the filtered cellular suspension is diluted to an appropriate cell density using a nutrient solution, which may be anything from a basic salt solution to a more complex nutrient solution.

Also disclosed is a cell suspension produced according to the above method. Preferably the cells in the suspension are autologous cells (i.e. they are isolated from the patient requiring an autograft). The inventors have observed that by removing xenogenic serum from the cell suspension there is less likelihood of transmission of infection and xenogenic reactions between a patient and the serum are eliminated. Another feature of the cell suspension produced according to the above method is that the tissue sample used to isolate the cells in the suspension is removed from the enzyme solution before the cells are harvested. When cells are exposed to enzymes capable of damaging inter-cellular adhesion the viability of the cellular suspension decreases over time thereby reducing the efficiency of the grafting when applied to a patient. The cell suspension produced according to the above method has been observed to possess greater cellular viability compared to comparative methods that harvest the cells at regular intervals whilst the tissue is immersed in the presence of enzymes like trypsin.

Also disclosed is a method of treating a patient in need of graft surgery, said method comprising the steps of:
(a) preparing an cell suspension according to the above method;
(b) administering the suspension directly to a region on the patient that requires a cell graft in a manner that facilitates spreading of the cell suspension in a relatively even distribution over the graft region.

In an alternate form, disclosed is the use of a cellular suspension suitable for grafts, which suspension is prepared according to the following steps:
(a) subjecting a tissue sample including cells suitable for grafting to a patient, to an enzyme suitable for dissociating cohesive pieces of the tissue stratum in the sample;
(b) removing the sample from the enzyme solution used in step (a) and harvesting in the presence of a nutrient solution cells from the tissue sample, which cells are suitable for grafting on to a patient wherein the nutrient solution is (i) free of xenogenic serum, (ii) capable of maintaining the viability of the cells until applied to a patient and (iii) is suitable for direct application to a region on a patient undergoing tissue grafting;
(c) filtering the cellular suspension produced according to step (b) to remove large cellular conglomerates;
for the preparation of medicament suitable for the treatment of tissue disorders requiring grafting.

According to the invention there is provided an apparatus for developing peri-operatively to a patient a cell suspension from a skin tissue sample, and for immediately applying the cell suspension to a graft site of the patient, comprising a first member (18), a second member (30), a set of tools required for cell harvesting and a spray nozzle for applying the cell suspension to the graft site, wherein:
(i) the first member (18) includes:
   (a) a heating means suitable for heating an enzyme solution to a required temperature and which is capable of maintaining that solution at the desired temperature for a suitable amount of time, wherein the pre-warmed enzyme solution is used to incubate a skin tissue sample and contains an amount of enzyme sufficient to dissociate cellular stratum in the skin tissue sample;
   (b) a filter recess (28) comprising a filter means (29) of a filter size between 50 µm and 200 µm, for removing cellular congregates from cells harvested from the skin tissue sample, to form the cell suspension that is suitable for immediate dispersion to the graft site;
   (c) at least a fluid containment well (26) for storage of a nutrient solution suitable for direct application to the graft site, wherein the nutrient solution is used to suspend the cells following release of the cells from the skin tissue sample; and
   (d) a storage compartment (36) for storing the set of tools required for cell harvesting; and
(ii) the second member (30) forms a reservoir (34) for withholding the skin tissue sample and the nutrient solution in fluid containment, wherein the reservoir is of sufficient size to permit manipulation of the skin tissue sample, permitting separation of the cellular stratum and harvesting of the cells from the stratum suitable for grafting;
wherein the first member (18) provides a seat (38) upon which the second member (30) may be placed during manipulation of the skin tissue sample.

When the enzyme solution is placed in contact with the heating means it is preferably heated in a manner that avoids localized heating within the solution.

Other aspects and advantages of the invention will become apparent to those skilled in the art from a review of the ensuing description, which proceeds with reference to the following illustrative drawings.

### BRIEF DESCRIPTION OF DRAWINGS

**Figure 1** illustrates a perspective view of the apparatus with the lid open and the second member in place.
**Figure 2** illustrates a perspective view of the apparatus with the lid open and the second member removed and inverted.
**Figure 3a** illustrates a perspective view of the first member of the apparatus.
**Figure 3b** illustrates a perspective rear view of the first member of the apparatus.
**Figure 4** illustrates a perspective view of the base of the apparatus.

### DETAILED DESCRIPTION OF THE INVENTION

### General

Those skilled in the art will appreciate that the invention described herein is susceptible to variations and modifications other than those specifically described. It is to be understood that the invention includes all such variation and modifications. The invention also includes all of the steps, features, compositions and compounds referred to or indicated in the specification, individually or collectively and any and all combinations or any two or more of the steps or features.

The present invention is not to be limited in scope by the specific embodiments described herein, which are intended for the purpose of exemplification only.

Throughout this specification and the claims that follow, unless the context requires otherwise, the word "comprise", or variations such as "comprises" or "comprising", will be understood to imply the inclusion of a stated integer or group of integers but not the exclusion of any other integer or group of integers.

### Description of Preferred Embodiments

Having regard to the above, there is disclosed a unique method and the invention provides a unique device suitable for producing a transplantable cellular suspension of living tissue suitable for grafting to a patient. In applying the method and/or in using the device donor tissue is harvested, subjected to a tissue dissociating means, cells suitable for grafting back to a patient are collected and dispersed in a solution that is suitable for immediate dispersion over the recipient graft site.

The subject invention has many advantages over the prior art some of which are illustrated in the following paragraphs.
1. It provides a time efficient method for supplying a cellular cover to a tissue in a clinical setting. That is, cells are available when needed at the time of surgery. This is achievable because there is a very short preparation period of the cells, thus allowing grafting to be performed peri-operatively or in the rooms of a specialist physician or General Practitioner.
2. It provides an apparatus, and there is disclosed a method, which significantly reduces the complexity associated with the use of conventional CEA's and is particularly useful in cases of burn injury that have presented late. In some instances, cells are unavailable at the time of surgery, either due to delayed referral of a patient with an unhealed burn or simply because the time needed for culturing of the grafts had exceeded that for preparation of the recipient would bed. The present invention ameliorates the issue of graft preparation time.
3. It aids in the achievement of rapid cell coverage in areas of injury and donor sites. It provides a means for reducing the size of donor sites - the biopsy donor site is markedly smaller than a split skin graft donor site and reduces or eliminates the use of split skin graft donor sites; improves expansion rate of cell coverage; improves the rate of healing of small burns; is useful for small areas of skin reconstructions, such as scars; and improves scar quality.
4. It ameliorates problems associated with the use of solutions used during conventional tissue culture process. According to the method of preparation and treatment the cells used in a graft are suspended in a nutrient solution free of xenogenic serum. That suspension is then placed directly onto the recipient site.
5. It provides a means for the treatment of various skin disorders or diseases. For example, it may be used for the following: epidermal resurfacing, replacement after skin loss, site match-up during re-pigmentation of an area of skin, treatment of burn wounds, leukoderma, vitiligo, piebaldism, in the treatment of scars - for example, caused through incorrect wound healing, improper scar direction or scar distortion from wound contraction, acne scars; resurfacing cosmetic dermabrasion, resurfacing after laser treatment and in association with dermal reconstruction. Additionally the method may be used for cell replacement therapy, including, for example, nerve cell replacement treatment, epithelial cell (such as urothelial cell, buccal mucosal cell and respiratory epithelial cell) replacement treatment, endothelial cell replacement treatment and osteogenic precursor cell replacement treatment.
6. It provides a means to produce a suspension of cells in a ratio to each other comparable with those seen in situ. That is, due to the manner of preparation of the cellular suspension, cells such as keratinocyte basal cells, Langerhans cells, fibroblasts and melanocytes typically have enhanced survival rates in comparison to standard tissue culture techniques, whereby selective cell culture can result in the loss of certain cell types. This has the advantage of allowing for the correct re-pigmentation of skin after a skin graft.
7. It allows faster surgery and healing - thereby reducing trauma for patients during the phase of their medical care.

There is disclosed a method for preparing a cell suspension suitable for use in resurfacing and regeneration of damaged tissue.

According to this method, tissue (preferably of an autologous nature) is harvested from a patient by means known in the art of tissue grafting. Preferably this is achieved by taking a tissue biopsy. With the harvesting of the biopsy consideration must be given to the depth of the biopsy and the surface area size. The depth and size of the biopsy influence the ease at which the procedure can be undertaken and speed with which a patient recovers from the procedure. Preferably the donor site should be chosen to appropriately match the recipient site, for example post-auricular for head and neck, thigh for lower limbs, inner-upper-arm for upper limbs, or palm for sole or vice-versa.

Once a biopsy has been harvested from a patient the tissue sample is subjected to physical and/or chemical dissociating means capable of dissociating cellular stratum in the tissue sample.

Methods for dissociating cellular layers within the tissues are well known in the field. For example, the dissociating means may be either a physical or a chemical disruption means. Physical dissociation means might include, for example, scraping the tissue sample with a scalpel, mincing the tissue, physically cutting the layers apart, or perfusing the tissue. Chemical dissociation means might include, for example, digestion with enzymes such as trypsin, dispase, collagenase, trypsin-EDTA, thermolysin, pronase, hyaluronidase, elastase, papain and pancreatin. Non-enzymatic solutions for the dissociation of tissue can also be used.

Preferably, dissociation of the tissue sample is achieved by placing the sample in a pre-warmed enzyme solution containing an amount of enzyme sufficient to dissociate cellular stratum in the tissue sample. This may be achieved, for example using a trypsin solution, however, any other enzyme such as dispase, collagenase, trypsin-EDTA, thermolysin, pronase, hyaluronidase, pancreatin, elastase and papain that cause cells to become detached from other cells or from solid surfaces may be used for this purpose. When the enzyme used is trypsin the enzyme solution used in the method is preferably calcium and magnesium free. One such solution is preferably calcium and magnesium ion free phosphate buffered saline.

Where the tissue biopsy is derived from a patient's skin (comprising epithelialdermal cells) the amount of trypsin that might be used in the method is preferably between about 5 and 0.1% trypsin per volume of solution. Desirable the trypsin concentration of the solution is about 2.5 to 0.25%, with about 0.5% trypsin being most preferred.

The time period over which the tissue sample is subjected to the trypsin solution may vary depending on the size of the biopsy sample taken. Preferably the tissue sample is placed in the presence of the trypsin solution for sufficient time to weaken the cohesive bonding between the tissue stratum. For example, where the tissue sample is taken from a patient's skin the sample might be placed in trypsin for a 5 to 60 minute period. Preferably, the tissue sample is immersed in the trypsin solution for between 10 and 30 minutes with 15 to 20 minutes being optimal for most tissue samples.

After the tissue sample has been immersed in the trypsin solution for an appropriate amount of time, the sample is removed from the trypsin and washed with nutrient solution. Washing the tissue sample may involve either partial or complete immersion of the treated sample in the nutrient solution. Alternatively, and more preferably, the wash solution is dripped on the tissue sample in sufficient volume to remove and or significantly dilute any excess trypsin solution from the surface of the sample. Preferably any dilution that might occur would lead to less than 0.05% trypsin in the nutrient solution.

The nutrient solution used in the method should be capable of significantly reducing and more preferably removing the effect of the trypsin either by dilution or neutralization. The nutrient solution used in the method will also preferably have the characteristics of being (i) free of at least xenogenic serum, (ii) capable of maintaining the viability of the cells until applied to a patient, and (iii) suitable for direct application to a region on a patient undergoing tissue grafting. The solution may be anything from a basic salt solution to a more complex nutrient solution. Preferably, the nutrient solution is free of all serum but contains various salts that resemble the substances found in body fluids; this type of solution is often called physiological saline. Phosphate or other non-toxic substances may also buffer the solution in order to maintain the pH at approximately physiological levels. A suitable nutrient solution that is particularly preferred is Hartmann's solution.

After application of the nutrient solution to the tissue sample, the cellular stratum of the sample are separated permitting cells capable of reproduction to be removed from the cellular material and suspended in the nutrient solution. Where the tissue sample is skin the dermis and epidermis are preferably separated to allow access to the dermal-epithelial junction of both surfaces.

Cells capable of reproduction are then removed from the separated stratum by any means known in the art. Preferably, the reproductive cells are scraped off the surface of the stratum using an instrument such as a scalpel. Cells capable of reproduction within the dermal-epithelial junction include but are not limited to keratinocyte basal cells, Langerhans cells, fibroblasts and melanocytes. Following release of the cells from the tissue sample they are suspended in the nutrient solution. Preferably only a small volume of nutrient solution is applied to the tissue sample during this harvesting step otherwise the suspension may become too fluid therein providing difficulties in applying the suspension to the graft.

To avoid excessively large cellular congregates in the cellular suspension the suspension is preferably filtered. Any filter capable of separating excessively large cellular congregates from the suspension may be used in this preferred step of the invention. In a highly preferred form of the invention the filter size is between 50 µm and 200 µm. More preferably it is between 75 µm and 150 µm, with 100 µm being one specific example.

Prior to application to the graft site or immediately after filtering, the cellular suspension may be diluted to produce an appropriate cell density suitable for the purpose to which the suspension is to be used.

Also disclosed is an aqueous cell suspension produced by the method described. The cell suspension provided by this method is highly suitable for tissue regeneration and grafting techniques. An important advantage of utilizing such a suspension in grafting technology is that it can be used to greatly expand the area or volume of a wound that can be treated quickly by in situ multiplication of a limited number of cells. The number and concentration of cells seeded onto graft site may be varied by modifying the concentration of cells in suspension, or by modifying the quantity of suspension that is distributed onto a given area or volume of the graft site.

By suspending cells in a nutrient solution which is at least (i) free of xenogenic serum, (ii) capable of maintaining the viability of the cells until applied to a patient and (iii) is suitable for direct application to a region on a patient undergoing tissue grafting, the inventors have found that the outcome of patient grafts is improved. A partial explanation for this appears to be attributable to the removal of xenogenic serum and more preferably all serum from the cell suspension. Xenogenic serum is a common additive in grafting culture medium and is well known to cause potential infective and hypersensitivity problems. Such serum is however generally required for the in *vitro* expansion of the cells and to neutralize the action of the enzyme if the enzyme used is trypsin. The nutrient solution used does not require such serum because the cell population within the suspension is not expanded prior to application to the graft site. Rather cellular multiplication is encouraged on the patient rather than in an *in vitro* system. When trypsin is used neutralization is achieved by other means.

Another unique feature of the cell suspension produced according to the disclosed method is that the composition of cells in the cellular preparation is comparable to that seen in situ compared to prior art cellular preparation. One possible explanation for this is that in the prior art, culture of the cellular preparation utilizes selective culture for keratinocytes, therefore loss of cellular constituents such as fibroblasts and melanocytes occurs whereas the cellular suspension produced from the disclosed method has a cell composition comparable to the in situ cell population. Another feature of the cellular suspension produced from the disclosed method is that the graft cells are more viable as they are harvested in a nutrient solution as distinct from prior art cell harvesting procedures which utilize techniques where the cells are harvested whilst exposed to powerful digestive enzymes for excessive periods of time. When the cells are exposed to such enzymes for excessive periods of time the viability of the cellular suspension decreases.

Also disclosed is a method of treatment of the patient requiring a tissue graft. By this method the cellular suspension produced according to the disclosed method is applied to a graft site. A liquid suspension containing cells may be manually distributed onto the graft site by any of several techniques, which include spraying, spreading, pipetting and painting.

Preferably the suspension is sprayed on to a graft site. The suspension may be sprayed through any type of nozzle that transforms liquid into small airborne droplets. This embodiment is subject to two constraints. First, it must not subject the cells in solution to shearing forces or pressures that would damage or kill substantial numbers of cells. Second, it should not require that the cellular suspension be mixed with a propellant fluid that is toxic or detrimental to cells or wound beds. A variety of nozzles that are commonly available satisfy both constraints. Such nozzles may be connected in any conventional way to a reservoir that contains the cellular suspension.

Alternatively the suspension may be delivered via a pipette, common "eye-droppers," syringe and needle and or other similar devices to place small quantities of cellular suspension on a graft site.

After the cell suspension has been applied to the recipient graft site, the wound may be dressed with a wound dressing. In a preferred embodiment the dressing is Surfasoft^{™} a woven nylon dressing. Preferably, the healing of the wound is followed up by standard protocols for skin graft treatment known to those skilled in the art.

According to the invention there is provided an apparatus for developing peri-operatively to a patient a cell suspension from a skin tissue sample, and for immediately applying the cell suspension to a graft site of the patient, comprising a first member (18), a second member (30), a set of tools required for cell harvesting and a spray nozzle for applying the cell suspension to the graft site, wherein:
(i) the first member (18) includes:
   (a) a heating means suitable for heating an enzyme solution to a required temperature and which is capable of maintaining that solution at the desired temperature for a suitable amount of time, wherein the pre-warmed enzyme solution is used to incubate a skin tissue sample and contains an amount of enzyme sufficient to dissociate cellular stratum in the skin tissue sample;
   (b) a filter recess (28) comprising a filter means (29) of a filter size between 50 µm and 200 µm, for removing cellular congregates from cells harvested from the skin tissue sample, to form the cell suspension that is suitable for immediate dispersion to the graft site;
   (c) at least a fluid containment well (26) for storage of a nutrient solution suitable for direct application to the graft site, wherein the nutrient solution is used to suspend the cells following release of the cells from the skin tissue sample and
   (d) a storage compartment (36) for storing the set of tools required for cell harvesting; and
(ii) the second member (30) forms a reservoir (34) for withholding the skin tissue sample and the nutrient solution in fluid containment, wherein the reservoir is of sufficient size to permit manipulation of the skin tissue sample permitting separation of the cellular stratum and harvesting of the cells from the stratum suitable for grafting;
wherein the first member (18) provides a seat (38) upon which the second member (30) may be placed during manipulation of the skin tissue sample.

In a preferred form of the invention the apparatus also includes a nutrient solution which solution is also capable of maintaining the viability of the cells in the tissue sample.

The fluid containment wells may alternatively serve as a receptacle for a container such as a plastic or glass vial that holds the nutrient solution. Preferably, the well is capable of holding at least a 10 ml volume. Such wells permit ease of fluid application to the tissue sample. Storage of such fluids in close proximity to its site of application also provides the advantage of reducing the risk of accidental leakage of the fluid and provides an easy means for accessing the fluid for accurately delivering it to either the tissue sample or the cell suspension.

The first member provides a storage compartment into which tools and solutions used in the above described method may be stored. Where such a compartment is provided in the apparatus the second member may provide the lid or closure to that compartment. In use the lid is preferably removed from the top of the compartment and inverted. The underside of the lid preferably forms the reservoir therein enabling the second member to serve a dual purpose. Tools and solutions used in the method can be accessed from the compartment. The inverted lid may then be seated back over the compartment therein providing the reservoir for the apparatus.

The apparatus may be made from metal, plastic or any other material. Further, the container may be any size. Preferably the size of the container is only limited by its intended use and the need for sterilization such as by the use of gamma irradiation and or ethylene oxide.

It should be appreciated that the heating means employed in the apparatus may simply constitute a heating pad or pads on the top of the first member. There are however, attendant problems with such arrangements, not least of which is the possibility of accidental spillage of the container undergoing heating. Therefore, in an embodiment, one or more heating means may be housed within a recess in the first member. Also located within that recess is at least a container into which tissue may be placed for exposure to the enzyme solution. In an alternate embodiment one or more heating means may be housed in the base of the apparatus. In such a configuration the first member contains at least an opening suitable for receiving a container capable of holding fluid, which opening provides access for the container to the heating means.

It will be appreciated that if the apparatus is designed for more than one use the heating means may be capable of being repeatedly heated and cooled. Alternatively each heating unit may be capable of a single use, but multiple heating units may be provided with the apparatus to facilitate multiple heating events.

In a highly preferred configuration of the apparatus a heating collar is located within a recess therein forming a heating recess in the first member within which there is located a container (e.g., a vial) for the enzyme. The container is preferably held in place by at least a restraining means, which desirably surrounds part of the upper portion of the container preventing accidental release of the container from the apparatus. In circumstances, where the apparatus is intended for single use the restraining means may be formed as an integral part of the first member, thus meaning that removal of the container may only be achieved by physically breaking the first member.

The heating means used in the apparatus is preferably controlled by circuitry permitting activation of the heating element when required. For example the heating means may be switched on by depressing the start button located, for example, on the surface of the first member. Alternately the heating means may be activated by pushing the container down with sufficient force to activate a switch located in the base of the apparatus. A person of ordinary skill in the field will appreciate that a wide range of electronic means may be used to activate the heating unit provided in the apparatus.

Desirably the heating unit is also operably linked to a timer mechanism, which is adapted to heat the enzyme solution for a pre-defined period of time. In circumstances where the apparatus is intended for multiple uses, preferably the timer can be set to deactivate the heating element when a particular amount of time is reached. At which point an alarm may activate to inform the user that the time is up. The alarm may be audible or in the form of a light display.

In a further preferred embodiment of the present invention the heating means may be provided with an adjustable temperature control. Where temperature adjustment is required such variation may be achieved by adapting the heating control circuitry to include or communicate with a temperature control mechanism permitting the temperature of the heating unit to be constantly varied within a constant range, or it may present a range of select temperatures that the heating control means can be set at. A temperature control means will beneficially be included in the apparatus where the apparatus is to be used in the harvesting and preparation of different cell types and or where different enzymes are used in the harvesting method for which the apparatus has unique application.

In an alternate more preferred form, the apparatus is designed for single use. In such instances the timer mechanism is part of the circuitry that controls the heating means. Once the heating means has been activated it heats the solution for a predefined period of time and then self-destructs. It should be appreciated by those skilled in the art that such an the apparatus may be fitted with various monitoring means that are capable of indicating such things as: the enzyme has reached the required temperature; the amount of time that the enzyme has been in the solution for; and or the amount of time left before the circuitry self-destructs etc. By way of example only, the monitoring means might consist of a series of LED's that activate when certain events occur. In a highly preferred embodiment the heating element preferably remains in the heating mode for a maximum of 45 minutes to 1 hour.

The heating means may be powered by any means known in the art. Preferably, the power supply is provided by battery/batteries. In one form of the invention, the power supply is a battery or a plurality of batteries located in the base of the apparatus.

In a further embodiment of the invention the apparatus may be provided with one or more means to facilitate mixing of the solutions used in the invention, such as for example an enzyme solution. In this respect, and by way of example only, the apparatus may include a means for vortexing the solution; such as an electromagnetic system that is adapted to agitate a magnetic bead. Where the apparatus includes an electromagnetic mixing system the magnetic bead is preferably provided in the container (e.g., vial) in which the solution is stored in the apparatus. Alternatively the magnetic bead may be added to the solution when mixing is desired.

In a highly preferred form of the invention the mixing means is combined with the heating means either as a single unit or as separate units to facilitate constant heating of the solution in an even manner. Using such a mixing means avoids the possible overheating of solution closest to the heating unit while the solution is heated. Such a system will provide a more constant heating of the solution. Alternate means for mixing the solution will be known in the art and include, mechanical, physical, electrical and electromagnetic means as an example. While any mixing means may be employed in the apparatus, preferably the mixing means is either selected to minimize vibration of the apparatus or incorporated into the apparatus in a manner that minimize such vibration. In this respect the mixing means may be housed on one or more vibration dampeners or the apparatus may include one or more vibration dampeners on is base.

Where a mixing means is incorporated into the apparatus the means may be automatically activated upon activation of the heating unit or alternatively there may be a separate activation system. Further, the speed of the mixing may either be fixed or variable. Preferably, there is a separate activation system for the mixing means.

The filter recess incorporated into the apparatus may be of any size or shape that facilitates filtering of a cellular suspension. Further the recess may be adapted to receive and hold at least a tube into which cell suspension may be filtered. Preferably the recess has a conical base providing easy means to access the full volume of cell suspension after it has been filtered.

Desirably, the third recess is designed to receive a 100 µm cell filter. The third recess can accommodate a 100 µm cell filter connected to a conical tube. Preferably, the tube has area/volume graduations marked on the side.

The apparatus includes a set of tools required for cell harvesting. It will be appreciated by those skilled in the art that any tools necessary for cell harvesting may be included with the device. Preferably, the set of tools are sterile. As an example only, the set of tools may include a glass vessel of separation enzyme; a sterile solution for suspension of the enzyme; a sterile nutrient solution; scalpel; forceps; syringe; medicine dropper, cell filter; wound dressings and/or spray nozzles. In a highly preferred embodiment, the set of tools are stored in a compartment formed in the first member of the apparatus, which is covered by the second member when not in use.

In a highly preferred embodiment, the device is used to harvest a suspension of cells and apply the cells to a recipient site in the following manner.

An aliquot of sterile water is mixed with a portion of lyophilized separation enzyme and placed in the heating recess. The heating means is then activated which heats the contents (i.e. the enzyme solution) of the container to a working temperature of between 30 and 37 °C, preferably between 33 and 37 °C and by way of example 37 °C within 2 minutes and maintains the working temperature for at least 45 minutes. Once an operational temperature has been reached, a sample of tissue taken from a donor site is placed in the enzyme solution and incubated at the working temperature. The tissue sample is incubated for between 5 to 45 minutes. Those skilled in the art would appreciate that the time taken to achieve separation of the layers of the tissue sample is dependent on the thickness and size of the tissue sample and the incubation temperature. Once enzymatic separation of the tissue layers is achieved, the tissue sample is removed to the reservoir and the tissue layers are separated using surgical instrument/s.

A carefully measured aliquot of the second solution is then withdrawn from the fluid containment well by aspiration into a syringe and then applied to the layers. The cells between the layers of tissue are scraped off and suspended by mixing with the nutrient solution. The cell suspension is then collected, preferably using a syringe and cannula.

The harvested suspension of cells is then passed through a cell filter located in the filter recess and the filtered suspension of cells is collected into the filter recess. The reservoir may optionally be rinsed with a further volume of the second solution and this resulting suspension of cells also filtered and collected in the filter recess.

The filtered suspension of cells may then optionally be aspirated into a syringe and applied to the recipient site.

### BEST MODE(S) FOR CARRYING OUT THE INVENTION

Further features of the present invention are more fully described in the following non-limiting Examples. It is to be understood, however, that this detailed description is included solely for the purposes of exemplifying the present invention. It should not be understood in any way as a restriction on the broad description of the invention as set out above.

### Example 1

### Preparation of Recipient Site

To optimize the success of the skin graft, the wound was cleaned and assessed to be of the appropriate depth. Further, blood haemostasis was established and the wound checked for evidence of surrounding cellulitis or infection. Techniques for preparing the area included sharp dissection, dermabrasion or laser-resurfacing.

### Donor Site Biopsy

The donor site was chosen to appropriately match the recipient site. The donor site was infiltrated with local anaesthetic and adrenaline underneath the skin near the subcutaneous tissue. This allowed the donor site to be firm and aided in the taking a thin split-thickness biopsy. The dimensions of the biopsy were determined by the size of the surface area of the recipient site to be covered.

Typically, the biopsy size has an expansion ratio of 1:10 - 1:80. In this case, a biopsy size of 2 cm x 2 cm was taken from the donor site giving an expansion ratio of 1:60.

### Cell Resurfacing Using the Rapid Technique

Treatment of the wound was carried out using the Re-Cell^{®} Rapid Technique cell harvesting apparatus, which is explained in more detail in Example 2 below. The apparatus contained all the instruments, solutions, enzymes and dressings required for wound treatment.

The heating element was activated by depressing the "start button". Solution (sterile water for injection) (10 ml) was transferred from the supplied plastic vessel marked Solution A into a glass vessel containing the separation enzyme (lyophilized trypsin) to give a final concentration of 0.5% trypsin. The enzyme solution was then mixed together, transferred to a vessel already located in the heating element recess and heated to 37 °C.

The vessel containing Solution B (nutrient media) was transferred from its supplied vessel into the fluid containment well.

The previously obtained tissue sample was then placed in the enzyme solution and incubated at 37 °C for between 10 to 15 minutes. After this time, the tissue sample was removed from enzyme solution with a pair of forceps, rinsed by dipping into the fluid containment well containing Solution B and placed with the dermal side down and the epidermal side up in the reservoir.

Solution B was then aspirated from the well into a syringe and dripped from the syringe onto both layers of the biopsy.

The skin layers were separated using forceps. This allowed access to the zone of the dermal-epidermal junction of both surfaces. Cells were scraped from the surfaces to develop a plume of cells in the reservoir. The cells were then mixed in Solution B. The plume of cells was then drawn up into the syringe via a 19 gauge cannula.

The supplied filter (100 µm cell filter) was mounted in the filter recess and the plume of cells in Solution B was passed through the filter. A further small amount of Solution B was then used to rinse the reservoir (e.g., a petri dish) and collect any remaining cells, which were also passed through the filter.

The resulting suspension of cells collected in the conical recess was aspirated into a syringe and a nozzle was attached to the syringe for spraying or dripping on to the wound area.

The wound was re-checked to ensure that it was clean and free of debris and that there was no evidence of bacterial contamination. Further, the wound was checked to determine if haemostasis had been achieved. Once the recipient site was ready, the suspension of cells was applied to the wound surface using the nozzle.

The wound was dressed with Surfasoft^{™} a woven nylon dressing, which was supplied with the apparatus. The healing of the wound was followed up using standard protocols for skin-graft treatment.

### Example 2

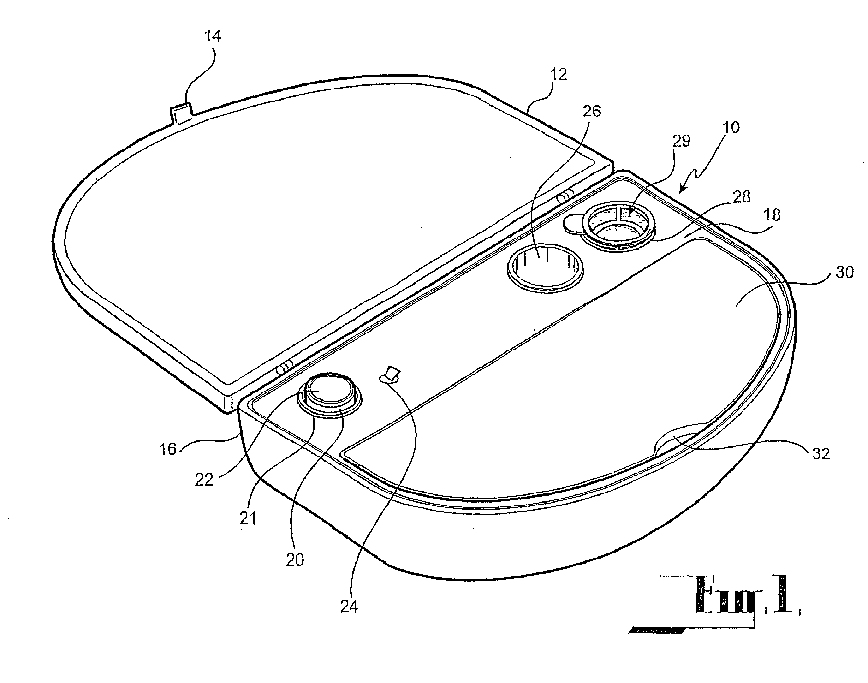

The embodiment shown in Figure 1 is directed to a Re-Cell^{®} Rapid Technique cell harvesting apparatus 10 for use in producing a transplantable cellular suspension of living tissue suitable for grafting to a patient.

As illustrated in figure 1 the apparatus includes a closure lid 12 possessing a locking mechanism 14 adapted to releasably engage a base portion 16. The locking mechanism 14 provides a means for closing the apparatus 16 when not in use. Located within the base portion 16 is a first member 18 within which there is provided an aperture 20 in which there is located a vial 22 for the enzyme. Adjacent the aperture there is provided an activation switch 24 capable of activating the heating means (not shown). The first member also provides a fluid containment well 26 and a filter recess 28. As presented in this illustration the filter 29 is shown located in the filter recess. Ordinarily the filter is an optional item included as a separate item in the apparatus.

The aperture 20 in the first member 18 is desirably of such a diameter that it allows the neck of the vial 22 to protrude through and above the first member 18. The periphery of the aperture 20 is fitted with a collar 21 which is slightly smaller than the diameter of the body of the vial 22. Thus, when in use, the vial 22 cannot be removed from the apparatus 10 as it is held in place by the collar 21 located around the periphery of the aperture 20.

Located adjacent to the aperture 20, fluid containment well 26 and filter recess 28 is the second member 30 which is positioned on a seat (not shown) located within a storage compartment (not shown) within the first member 18. When inverted the second member 30 forms a reservoir within which tissue manipulations may be performed. To facilitate separation of the second member 30 from the first member 18 an indent 32 is provided in the side of a portion of the second member 30, which is of such a size that a person can lift the second member from the seat on which it resides in the first member 18.

Within the filter recess 28 there is located a filter 29 (provided separately with the other components) having a mesh therein capable of separating cellular material of greater than 100 µm from a cell supernatant.

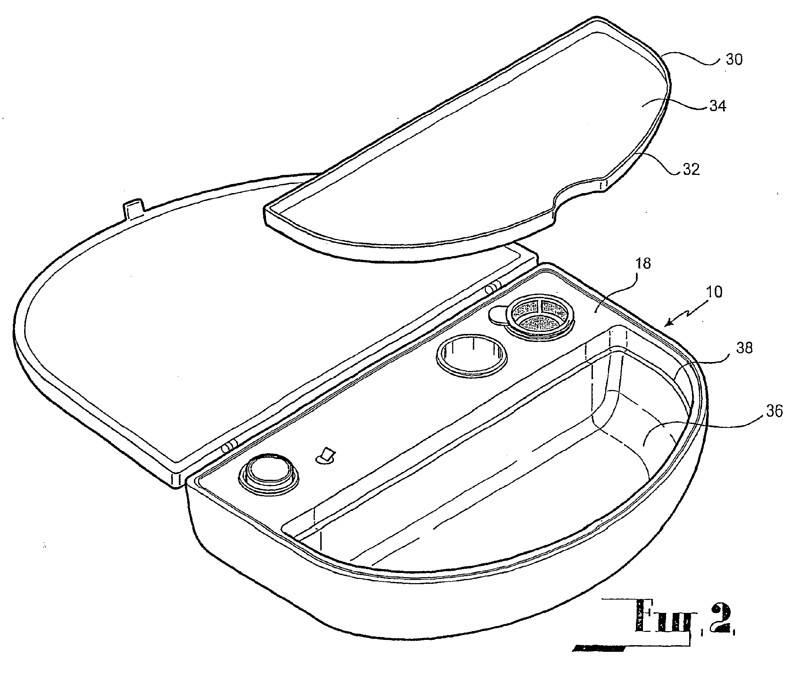

Figure 2 provides a partially exploded perspective view of the apparatus 10 wherein the second member 30 is removed from the first member 18 and inverted. Inversion of the second member 30 reveals the sidewalls 32 of the second member 30, which form the fluid containment barrier of the reservoir and a reservoir area 34 in which tissue manipulations may be performed.

Removal of the second member 30 from the first member 18 also reveals a storage compartment 36 in the first member 18 in which solutions and tools may be stored when the apparatus 10 is not in use. Within the storage compartment 36 there is located a seat 38 upon which the second member 30 may reside. The seat 38 is preferably located around the periphery of the storage compartment 36 at a depth beneath the surface of the first member 18 that is equivalent to the height of the sidewalls 32 of the second member 30.

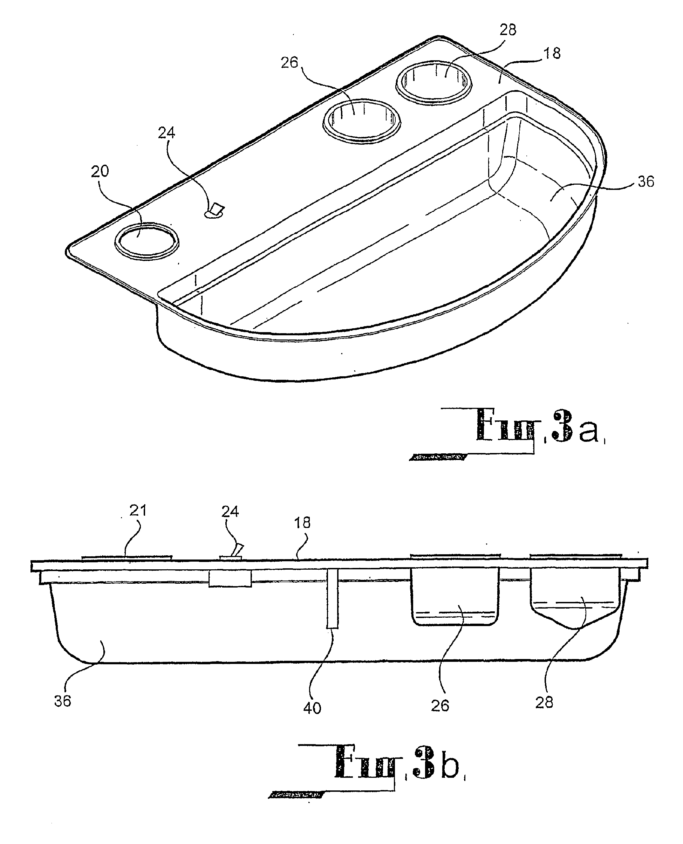

Figure 3a provides a perspective view of the first member 18 showing the storage compartment 36, the heater activating switch 24, the aperture 20, the fluid containment well 26 and filter recess 28 formed within the first member. Figure 3b provides a rear view of the first member 18 showing the filter recess 28, the fluid containment well 26, the heater activating switch 24, the aperture collar 21 and the rear wall of the storage compartment 36. As seen in this figure the filter recess has a conical base thereby providing a means for easy access to the cell suspension that is filtered into it. Located adjacent to the fluid containment well and on the opposite side of the filter containment well there is also provided a battery positioning member 40 which protrudes towards the base 16 of the apparatus (not shown) and provides a means for holding the batteries in place, which are required for activating the heating means.

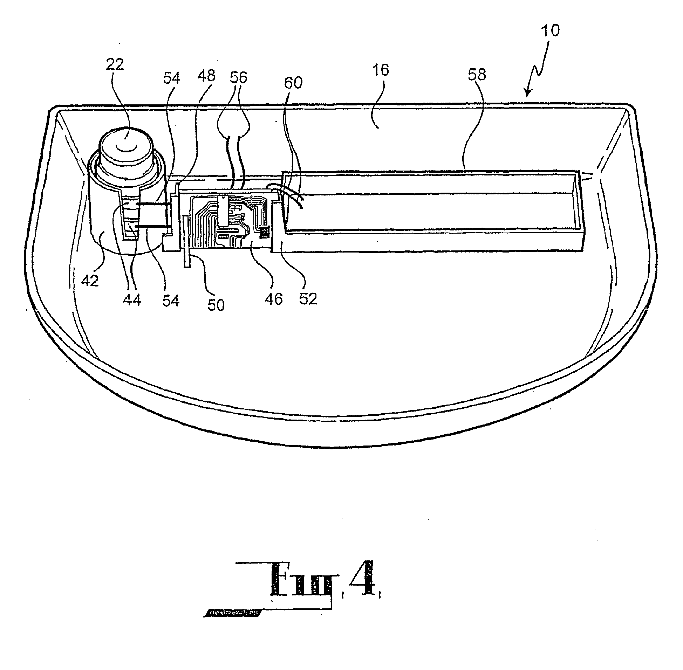

Figure 4 provides a perspective view of the base 16 of the apparatus 10 showing the vial 22 located within a containment field 42. Between the containment field 42 and the vial 22 there is located a heating collar(s) 44, which surrounds the body of the vial. Adjacent the containment field there is a circuit board 46, which is held in position by circuit board containment means 48, 50 and 52. Said circuit board 46 is in electrical communications with the heating collar(s) 44 by wires 54. The circuit board is also in electrical communication via wires 58 with the heater activating switch (not shown). Adjacent the circuit board 46 there is provided a battery containment means 58, which holds 4 AA batteries in immovable position (not shown). The batteries are in electrical communication with the circuit board 46 by wires 60. When the first member 18 is fitted to the base 16 the batteries are held in place by the battery containment means 58, the battery positioning means 40 and the base of each of the fluid containment well 26 and the filter recess 28. Preferably the conical base of the filter recess 28 also protrudes between the batteries therein providing a further means for securing the batteries in immovable position.

Modifications and variations of the described methods and device of the invention will be apparent to those skilled in the art without departing from the scope of the invention. Although the invention has been described in connection with specific preferred embodiments, it should be understood that the invention as claimed should not be unduly limited to such specific embodiments.

## Claims

1. An apparatus for developing peri-operatively to a patient a cell suspension from a skin tissue sample, and for immediately applying the cell suspension to a graft site of the patient, comprising a first member (18), a second member (30), a set of tools required for cell harvesting and a spray nozzle for applying the cell suspension to the graft site, wherein:
(i) the first member (18) includes:
(a) a heating means suitable for heating an enzyme solution to a required temperature and which is capable of maintaining that solution at the desired temperature for a suitable amount of time, wherein the pre-warmed enzyme solution is used to incubate a skin tissue sample and contains an amount of enzyme sufficient to dissociate cellular stratum in the skin tissue sample;
(b) a filter recess (28) comprising a filter means (29) of a filter size between 50 µm and 200 µm, for removing cellular congregates from cells harvested from the skin tissue sample, to form the cell suspension that is suitable for immediate dispersion to the graft site;
(c) at least a fluid containment well (26) for storage of a nutrient solution suitable for direct application to the graft site, wherein the nutrient solution is used to suspend the cells following release of the cells from the skin tissue sample; and
(d) a storage compartment (36) for storing the set of tools required for cell harvesting; and
(ii) the second member (30) forms a reservoir (34) for withholding the skin tissue sample and the nutrient solution in fluid containment, wherein the reservoir is of sufficient size to permit manipulation of the skin tissue sample, permitting separation of the cellular stratum and harvesting of the cells from the stratum suitable for grafting;
wherein the first member (18) provides a seat (38) upon which the second member (30) may be placed during manipulation of the skin tissue sample.

2. The apparatus of claim 1, wherein the heating means is housed within a recess in the first member.

3. The apparatus of claim 2, wherein the recess further comprises a container located therein into which the skin tissue sample may be placed for exposure to the enzyme solution.

4. The apparatus of any one of claims 1-3, wherein the heating means is controlled by circuitry permitting activation of the heating means when required.

5. The apparatus of any one of claims 1-4, wherein the heating means is operably linked to a timer mechanism adapted to heat the enzyme solution for a pre-defined period of time.

6. The apparatus of any one of claims 1-5, wherein the heating means is provided with an adjustable temperature control.

7. The apparatus of any one of claims 1-6, further comprising a monitoring means that are capable of indicating that the enzyme has reached the required temperature, and/or the amount of time that the enzyme has been in the enzyme solution for.

8. The apparatus of any one of claims 1-7, further comprising a means to facilitate mixing of the enzyme solution.

9. The apparatus of any one of claims 1-8, wherein the filter recess is adapted to receive and hold at least a tube into which the cell suspension may be filtered.

10. The apparatus of any one of claims 1-9, wherein the fluid containment well serves as a receptacle for a container that holds the nutrient solution.

11. The apparatus of any one of claims 1-10, wherein the second member provides a lid to the storage compartment.

12. The apparatus of claim 11, wherein the underside of the lid forms the reservoir therein enabling the second member to serve a dual purpose.

13. The apparatus of any one of claims 1-12, wherein the set of tools include a glass vessel of the enzyme, a sterile solution for suspension of the enzyme, a sterile nutrient solution, scalpel, forceps, syringe, medicine dropper, cell filter, wound dressings and/or spray nozzles.

14. The apparatus of any one of claims 1-13, wherein the heating means heats the enzyme solution to a working temperature of between 30 and 37 °C, preferably between 33 and 37 °C.

15. The apparatus of claim 14, wherein the heating means maintains the working temperature for at least 45 minutes.

## Patentansprüche

1. Ein Apparat zur perioperativen Entwicklung einer Zellsuspension aus einer Hautgewebeprobe für einen Patienten und zur unmittelbaren Anwendung der Zellsuspension an einer Transplantatstelle des Patienten, bestehend aus einem ersten Teil (18), einem zweiten Teil (30), einem Werkzeugsatz, der für die Zellernte notwendig ist, und einer Sprühdüse zur Anwendung der Zellsuspension an der Transplantatstelle, wobei:
(i) das erste Teil (18) einschließt:
(a) ein Heizelement zum Erhitzen einer Enzymlösung auf eine erforderliche Temperatur, das in der Lage ist, diese Lösung für eine entsprechende Zeit in der gewünschten Temperatur zu halten, wobei die vorgewärmte Enzymlösung verwendet wird, um eine Hautgewebeprobe zu inkubieren, und eine ausreichende Menge an Enzymen enthält, um das Zellstratum in der Hautgewebeprobe zu dissoziieren;
(b) eine Filtervertiefung (28), bestehend aus einem Filterelement (29), mit einer Filtergröße zwischen 50 um und 200 um, um Zellkollektive aus Zellen zu entnehmen, die aus der Hautgewebeprobe geerntet wurden, um die Zellsuspension zu bilden, die für die unmittelbare Verteilung an der Transplantatstelle geeignet ist;
(c) mindestens ein Flüssigkeitsbehälter (26) für die Aufbewahrung einer Nährlösung zur direkten Anwendung an der Transplantatstelle, wobei die Nährlösung dazu verwendet wird, die Zellen nach der Zellfreisetzung aus der Hautgewebeprobe zu suspendieren; und
(d) ein Ablagebehälter (36) zum Ablegen des Werkzeugsatzes, der für die Zellernte gebraucht wird; und
(ii) das zweite Teil (30) bildet einen Sammelbehälter (34) zur Aufbewahrung der Hautgewebeprobe und der Nährlösung im Flüssigkeitsbehälter, wobei der Sammelbehälter ausreichend groß ist, um eine Verarbeitung der Hautgewebeprobe und die Trennung des Zellstratum und das Ernten der für die Transplantation geeigneten Zellen aus dem Stratum zu erlauben;
wobei das erste Teil (18) eine Auflagefläche (38) hat, auf die das zweite Teil (30) während der Verarbeitung der Hautgewebeprobe aufgesetzt werden kann.

2. Der Apparat in Anspruch 1, wobei das Heizelement in einer Vertiefung im ersten Teil untergebracht ist.

3. Der Apparat in Anspruch 2, wobei die Vertiefung darüberhinaus einen darin enthaltenen Behälter aufweist, in den die Hautgewebeprobe eingelegt werden kann, um sie mit der Enzymlösung in Verbindung zu bringen.

4. Der Apparat in einem der Ansprüche 1-3, wobei das Heizelement von einer Schalttechnik gesteuert und, sobald erforderlich, die Aktivierung des Heizelementes ermöglicht wird.

5. Der Apparat in einem der Ansprüche 1-4, wobei das Heizelement funktionsfähig mit einem Zeitschaltmechanismus verbunden ist, der dazu dient, die Enzymlösung über einen vorbestimmten Zeitraum zu erwärmen.

6. Der Apparat in einem der Ansprüche 1-5, wobei das Heizelement mit einer einstellbaren Temperaturkontrolle ausgestattet ist.

7. Der Apparat in einem der Ansprüche 1-6, darüberhinaus bestehend aus einer Überwachungsvorrichtung, die anzeigt, dass das Enzym die erforderliche Temperatur erreicht hat, und/oder den Zeitraum anzeigt, in dem sich das Enzym in der Enzymlösung befand.

8. Der Apparat in einem der Ansprüche 1-7, darüberhinaus bestehend aus einer Vorrichtung zum Mischen der Enzymlösung.

9. Der Apparat in einem der Ansprüche 1-8, wobei die Filtervertiefung so gestaltet ist, dass sie mindestens einen Schlauch aufnehmen und enthalten kann, in den die Zellsuspension gefiltert wird.

10. Der Apparat in einem der Ansprüche 1-9, wobei der Flüssigkeitsbehälter als Aufnahme für einen Behälter dient, der die Nährlösung enthält.

11. Der Apparat in einem der Ansprüche 1-10, wobei das zweite Teil einen Deckel am Ablagebehälter aufweist.

12. Der Apparat in Anspruch 11, wobei die Unterseite des Deckels einen Sammelbehälter bildet, durch den das zweite Teil einen dualen Verwendungszweck erhält.

13. Der Apparat in einem der Ansprüche 1-12, wobei der Werkzeugsatz ein Glasgefäß des Enzyms, eine sterile Lösung zur Enzymsuspension, eine sterile Nährlösung, Skalpell, Zange, Injektionsspritze, Pasteurpipette, Zellfilter, Wundverbände und/oder Sprühdüsen enthält.

14. Der Apparat in einem der Ansprüche 1-13, wobei das Heizelement die Enzymlösung auf eine Arbeitstemperatur zwischen 30 und 37 °C, vorzugsweise aber zwischen 33 und 37 °C, erwärmt.

15. Der Apparat in Anspruch 14, wobei das Heizelement die Arbeitstemperatur mindestens für 45 Minuten aufrechterhält.

## Revendications

1. Un appareil de développement de manière péri-opératoire chez un patient d'une suspension de cellules à partir d'un échantillon de tissu cutané et d'application immédiate de la suspension de cellules à un site de greffe du patient, comprenant un premier élément (18), un deuxième élément (30), un ensemble d'outils nécessaires à une récolte de cellules et une buse de pulvérisation destinée à l'application de la suspension de cellules au site de greffe, où :
(i) le premier élément (18) comprend :
(a) un moyen de chauffage adapté au chauffage d'une solution enzymatique à une température requise et qui est capable de maintenir cette solution à la température souhaitée pendant une durée adaptée, où la solution enzymatique préchauffée est utilisée pour incuber un échantillon de tissu cutané et contient une quantité d'enzyme suffisante pour dissocier une strate cellulaire dans l'échantillon de tissu cutané,
(b) un évidement pour filtre (28) comprenant un moyen de filtrage (29) d'une taille de filtre située entre 50 µm et 200 µm destiné à éliminer des agrégats cellulaires des cellules récoltées à partir de l'échantillon de tissu cutané de façon à former la suspension de cellules qui est adaptée à une dispersion immédiate sur le site de greffe,
(c) au moins un puits de rétention de fluide (26) destiné au stockage d'une solution nutritive adaptée à une application directe sur le site de greffe, où la solution nutritive est utilisée pour suspendre les cellules après libération des cellules de l'échantillon de tissu cutané, et
(d) un compartiment de stockage (36) destiné au stockage de l'ensemble d'outils nécessaires à une récolte de cellules, et
(ii) le deuxième élément (30) forme un réservoir (34) destiné à retenir l'échantillon de tissu cutané et la solution nutritive en confinement fluidique, où le réservoir est d'une taille suffisante pour permettre une manipulation de l'échantillon de tissu cutané, permettant la séparation de la strate cellulaire et la récolte des cellules à partir de la strate adaptée à une greffe,
où le premier élément (18) fournit un siège (38) sur lequel le deuxième élément (30) peut être placé au cours d'une manipulation de l'échantillon de tissu cutané.

2. L'appareil selon la Revendication 1, où le moyen de chauffage est logé à l'intérieur d'un évidement dans le premier élément.

3. L'appareil selon la Revendication 2, où l'évidement comprend en outre un récipient situé dans celui-ci dans lequel l'échantillon de tissu cutané peut être placé pour exposition à la solution enzymatique.

4. L'appareil selon l'une quelconque des Revendications 1 à 3, où le moyen de chauffage est commandé par un circuit permettant une activation du moyen de chauffage lorsque nécessaire.

5. L'appareil selon l'une quelconque des Revendications 1 à 4, où le moyen de chauffage est relié de manière opérationnelle à un mécanisme d'horloge adapté de façon à chauffer la solution enzymatique pendant une période temporelle prédéfinie.

6. L'appareil selon l'une quelconque des Revendications 1 à 5, où le moyen de chauffage est équipé d'un régulateur de température ajustable.

7. L'appareil selon l'une quelconque des Revendications 1 à 6, comprenant en outre un moyen de surveillance qui est capable d'indiquer que l'enzyme a atteint la température requise et/ou la durée pendant laquelle l'enzyme est resté dans la solution enzymatique.

8. L'appareil selon l'une quelconque des Revendications 1 à 7, comprenant en outre un moyen de favoriser le mélange de la solution enzymatique.

9. L'appareil selon l'une quelconque des Revendications 1 à 8, où l'évidement pour filtre est adapté de façon à recevoir et contenir au moins un tube dans lequel la suspension de cellules peut être filtrée.

10. L'appareil selon l'une quelconque des Revendications 1 à 9, où le puits de rétention de fluide sert de réceptacle à un récipient qui contient la solution nutritive.

11. L'appareil selon l'une quelconque des Revendications 1 à 10, où le deuxième élément fournit un couvercle au compartiment de stockage.

12. L'appareil selon la Revendication 11, où la partie inférieure du couvercle forme le réservoir de celui-ci, permettant au deuxième élément de servir un double objectif.

13. L'appareil selon l'une quelconque des Revendications 1 à 12, où l'ensemble d'outils comprend un récipient en verre de l'enzyme, une solution stérile pour la suspension de l'enzyme, une solution stérile nutritive, un scalpel, des forceps, une seringue, un compte-gouttes de médicament, un filtre de cellules, des pansements et/ou des buses de pulvérisation.

14. L'appareil selon l'une quelconque des Revendications 1 à 13, où le moyen de chauffage chauffe la solution enzymatique à une température de travail située entre 30 et 37°C, de préférence entre 33 et 37°C.

15. L'appareil selon la Revendication 14, où le moyen de chauffage maintient la température de travail pendant au moins 45 minutes.
